# EUROPEAN PATENT APPLICATION

(11) **EP 4 527 468 A1**
(43) Date of publication of application: **26.03.2025**
(21) Application number: 23839343.3
(22) Date of filing: 01.06.2023
(51) Int. Cl.: A61Q 1/00, A61K 8/02, A61K 8/73

(54) **SOLID POWDER COSMETIC**

(30) Priority: 13.07.2022 JP 2022112171
(71) Applicant: Sano Co., Ltd., Osaka-shi, Osaka 530-0005 (JP); Daio Paper Corporation, Ehime 799-0492 (JP)
(72) Inventor: SASAKI, Hiroto, Shikokuchuo-shi, Ehime 799-0492 (JP); MIDORIKAWA, Yuto, Yokohama-shi, Kanagawa 233-0002 (JP)
(74) Representative: Meissner Bolte Partnerschaft mbB
(86) International application number: PCT/JP2023/020501
(87) International publication number: WO 2024/014166

(57) **Abstract**

To provide a solid powder cosmetic that is smooth and is resistant to impact.

The problem is solved by a solid powder cosmetic containing a cellulose powder having an average particle size of 1 to 25 µm and an aspect ratio of 0.6 to 0.9, wherein the cellulose powder is an aggregate containing an additive and fine fibrous cellulose having an average fiber width of 1 to 500 nm.

## Description

### Technical Field

The present invention relates to a solid powder cosmetic.

### Background Art

Conventionally, a solid powder cosmetic such as a foundation or an eye shadow is manufactured by a press molding method in which a cosmetic composition is filled into a container and compressed to be solidified. The solid powder cosmetic has a relatively small blending amount of an oil component, and therefore has a week bonding force between particles of the powder and is hardly solidified. Therefore, molding is performed by increasing a pressing pressure, but a molded product manufactured in this way is hard and has a powdery touch.

Therefore, in the solid powder cosmetic, an elastic spherical resin powder such as a silicone elastomer or polyurethane may be used such that a moist feeling and a smooth and soft touch can be obtained. However, when a large amount of the resin powder is blended, the solid powder cosmetic has poor moldability and easily cracks by impact disadvantageously. There has been a certain difficulty in manufacturing a solid powder cosmetic having both smoothness and impact resistance as described above.

In Patent Literature 1, the above-described difficulty is solved by using 30 to 90% by weight of an organic powder made of an acylated taurine polyvalent metal salt having an average crushing strength of 0.2 to 1 kg/mm² and a film-forming polymer having an elastic modulus of 1 to 100 kg/cm². Patent Literature 2 focuses on an oil-soluble rice bran extract having excellent moisture retention, and proposes a powder cosmetic in which an oil-soluble rice bran extract is blended in an oil agent contained in the powder cosmetic at a specific ratio. Although this powder cosmetic is a powder, the powder cosmetic is smooth without providing a friction feeling at the time of application, provides a specific touch such that the powder is stuck to the skin and the skin after application is slippery and soft, and reduces cracking due to falling. Patent Literature 3 proposes a makeup cosmetic containing cellulose granules.

### Citation List

### Patent Literature

Patent Literature 1: JP 2002-80325 A
Patent Literature 2: JP 2021-98674 A
Patent Literature 3: WO 2018/194050 A

### Summary of Invention

### Technical Problem

The techniques described in Patent Literatures 1 and 2 focus not on a powder contained in a solid powder cosmetic but on a component other than a powder, for example, a film-forming polymer and an oil-soluble rice bran extract. By mixing these components, impact resistance and a smooth touch are imparted to the solid powder cosmetic. Meanwhile, the inventors of the present invention have focused on a powder, and have made studies on a powder having impact resistance and a smooth touch by inclusion of the powder in a solid powder cosmetic.

### Solution to Problem

As a result of intensive studies, the present inventors have found that a solid powder cosmetic containing a cellulose powder solves the above-described problems, and aspects of a completed invention are as follows. Note that the cellulose granules described in Cited Literature 3 are different from the cellulose powder of the present invention.

### (First Aspect)

A solid powder cosmetic containing
a cellulose powder having an average particle size of 1 to 25 µm and an aspect ratio of 0.6 to 0.9,
wherein the cellulose powder is an aggregate containing an additive and fine fibrous cellulose having an average fiber width of 1 to 500 nm.

The cellulose powder contained in the above-described solid powder cosmetic contains fine fibrous cellulose. Since the fine fibrous cellulose forms an elongated fiber as can be seen from its name, the cellulose powder containing the fine fibrous cellulose is not spherical but has a shape distorted from a spherical shape. The distorted shape can be expressed, for example, in an aspect ratio within the above-described range. A solid powder cosmetic containing a cellulose powder having an average particle size within the above-described range and an aspect ratio within the above-described range can have both resistance to impact (impact resistance) and a use feeling such as smoothness, a moist feeling, or a soft touch. A reason why the solid powder cosmetic containing the cellulose powder can have both impact resistance and a use feeling is probably as follows.

In order to improve hardness and powdery properties which are drawbacks of a conventional solid powder cosmetic, blending of elastic spherical particles has been studied. However, the elastic spherical particles are soft, have a spherical rolling effect, have a moist touch, and smoothly spread, but are elastically deformed when being compression-molded. Therefore, force to return to an original state acts when pressure is released after molding, and therefore it is difficult to mold the elastic spherical particles, and the elastic spherical particles have poor impact resistance after molding.

On the other hand, it is presumed that cellulose particles used in the present invention are plastically deformed when being compressed, thereby increasing a contact area between the particles and improving moldability and impact resistance of the solid powder cosmetic. In addition, even if there is no deformation due to pressure, the cellulose powder used in the present invention includes those having a distorted shape and has a larger specific surface area than general spherical particles, and the powder is non-spherical. Therefore, it is assumed that when the cellulose powder is mixed with components of a cosmetic, particles of the cellulose powder are arranged in a random direction, and the number of contact points between the components increases, whereby cracking is reduced due to reduced external force to impact resistance.

In addition, when a dispersion of fine fibrous cellulose is dried as it is, particles in which fibers are firmly and randomly bonded to each other by hydrogen bonding are obtained. On the other hand, in a cellulose powder containing fine fibrous cellulose and an additive (for example, a hydrophilic humectant) during drying, the additive moderately inhibits formation of hydrogen bonding between cellulose fibers to add softness. Therefore, it is presumed that a moist touch is obtained, and an effect of improving moldability by plastic deformation is also obtained.

In addition to the first aspect, the following aspects are also a preferred aspects.

### (Second Aspect)

The solid powder cosmetic according to the first aspect,
wherein the cellulose powder is formed by aggregation of the fine fibrous cellulose.

### (Third Aspect)

The solid powder cosmetic according to the first aspect, containing:
60 to 99.9% by mass of a powder containing the cellulose powder; and 0.1 to 40% by mass of an oil component.

### (Fourth Aspect)

The solid powder cosmetic according to the first aspect,
wherein the additive is glycerin.

### (Fifth Aspect)

The solid powder cosmetic according to the first aspect,
wherein the cellulose powder has a moisture content of 1 to 15% in an atmosphere having a humidity of 50%.

### (Sixth Aspect)

The solid powder cosmetic according to the first aspect,
wherein the cellulose powder has a bulk specific gravity of 0.5 g/cm³ or less.

### (Seventh Aspect)

The solid powder cosmetic according to the first aspect,
wherein the cellulose powder has a specific surface area of 1.0 m²/g or more.

### (Eighth Aspect)

The solid powder cosmetic according to the first aspect, containing
1 to 99% by mass of the cellulose powder

### (Ninth Aspect)

The solid powder cosmetic according to the first aspect,
wherein the cellulose powder has a cellulose pulp viscosity of 1.0 to 7.0 mPa•s.

### (Tenth Aspect)

The solid powder cosmetic according to the first aspect, which is a base makeup cosmetic.

### Advantageous Effects of Invention

The present invention provides a smooth solid powder cosmetic having resistance to impact (impact resistance).

### Brief Description of Drawings

Fig. 1 is an SEM image of a cellulose powder A.
Fig. 2 is an SEM image of a cellulose powder B.
Fig. 3 is an SEM image of a cellulose powder C.
Fig. 4 is an SEM image of a cellulose powder D.
Fig. 5 is an SEM image of a silicone powder.
Fig. 6 is an SEM image of a silica powder.

### Description of Embodiments

Next, an embodiment for carrying out the invention will be described. Note that the present embodiment is an example of the present invention. The scope of the present invention is not limited to the scope of the present embodiment.

A solid powder cosmetic of the present embodiment contains a cellulose powder having an average particle size of 1 to 25 µm and an aspect ratio of 0.6 to 0.9, wherein the cellulose powder is an aggregate containing an additive and fine fibrous cellulose having an average fiber width of 1 to 500 nm. The composition of the solid powder cosmetic will be described below.

### (Fine fibrous cellulose)

The cellulose powder contained in the solid powder cosmetic of the present embodiment contains fine fibrous cellulose (also referred to as "CNF"), and the fine fibrous cellulose can be obtained by defibrating a raw material pulp (making a raw material pulp finer).

As the raw material pulp for the fine fibrous cellulose, one or more kinds can be selected from the group consisting of, for example, a wood pulp made from hardwood, softwood, or the like, a non-wood pulp made from straw, bagasse, cotton, hemp, bast fiber, or the like, and a de-inked pulp (DIP) made from recovered used paper, waste paper, or the like to be used.

Note that it is preferable to use a wood pulp rather than a non-wood pulp or a de-inked pulp because mixing of impurities is avoided as much as possible, and α-cellulose insoluble in an alkali among cellulose components can be obtained at a high content. By treating the wood pulp with an alkali, components soluble in the alkali can be removed, and the purity of cellulose can be increased.

As the wood pulp, for example, one or more kinds can be selected from the group consisting of a chemical pulp such as a hardwood kraft pulp (LKP) or a softwood kraft pulp (NKP), and a mechanical pulp (TMP) to be used.

The hardwood kraft pulp may be a hardwood bleached kraft pulp, a hardwood unbleached kraft pulp, or a hardwood semi-bleached kraft pulp. Similarly, the softwood kraft pulp may be a softwood bleached kraft pulp, a softwood unbleached kraft pulp, or a softwood semi-bleached kraft pulp.

As the mechanical pulp, for example, one or more kinds selected from the group consisting of a stone ground pulp (SGP), a pressure stone ground pulp (PGW), a refiner ground pulp (RGP), a chemiground pulp (CGP), a thermoground pulp (TGP), a ground pulp (GP), a thermomechanical pulp (TMP), a chemithermomechanical pulp (CTMP), a refiner mechanical pulp (RMP), and a bleached thermomechanical pulp (BTMP) can be used. Note that, in order to avoid the above-described mixing of impurities other than cellulose, it is particularly preferable to use a chemical pulp such as a hardwood kraft pulp (LKP) or a softwood kraft pulp (NKP).

Prior to defibration into the fine fibrous cellulose, pretreatment can also be performed by a chemical method. Examples of the pretreatment by a chemical method include hydrolysis of a polysaccharide by an acid (acid treatment), hydrolysis of a polysaccharide by an enzyme (enzyme treatment), swelling of a polysaccharide by an alkali (alkali treatment), oxidation of a polysaccharide by an oxidizing agent (oxidation treatment), and reduction of a polysaccharide by a reducing agent (reduction treatment).

By performing an alkali treatment prior to the defibration, a part of hydroxyl groups of a hemicellulose or a cellulose included in the pulp is dissociated, and molecules are anionized to weaken intramolecular and intermolecular hydrogen bonds, resulting in promoting dispersion of cellulose fibers in the defibration.

As an alkali used for the alkali treatment, for example, sodium hydroxide, lithium hydroxide, potassium hydroxide, an aqueous ammonia solution, and an organic alkali such as tetramethylammonium hydroxide, tetraethylammonium hydroxide, tetrabutylammonium hydroxide, or benzyltrimethylammonium hydroxide can be used. Note that sodium hydroxide is preferably used from a viewpoint of manufacturing cost.

When an enzyme treatment, an acid treatment, or an oxidation treatment is performed prior to the defibration, the water retention degree of the fine fibrous cellulose can be lowered, the degree of crystallinity thereof can be increased, and homogeneity thereof can be increased. In this regard, when the water retention degree of the fine fibrous cellulose is low, the fine fibrous cellulose is easily dehydrated, and a dehydration property of a fine fibrous cellulose dispersion (hereinafter, also referred to as a "slurry") is improved.

When the raw material pulp is subjected to an enzyme treatment, an acid treatment, or an oxidation treatment, an amorphous region of hemicellulose or cellulose included in the pulp is decomposed. When the amorphous region is decomposed, energy required for making the pulp finer can be reduced, and homogeneity and dispersibility of cellulose fibers can be improved. When cellulose fibers have excellent homogeneity and dispersibility, a cellulose powder manufactured from fine fibrous cellulose tends to be homogeneous with little variation in particle size. When the variation in the particle size of the cellulose powder is large, there is a possibility that the cellulose powder is not sufficiently dispersed in the solid powder cosmetic and is unevenly distributed. Therefore, the degree of decomposition of the amorphous region of hemicellulose or cellulose in the pulp is one of important factors. Note that it is preferable to avoid excessive pretreatment because the pretreatment reduces an aspect ratio of the fine fibrous cellulose and manufactures a cellulose powder having an average particle size deviating from a desired average particle size.

The defibration of the raw material pulp can be performed by beating the raw material pulp using, for example, a beater, a homogenizer such as a high-pressure homogenizer or a high-pressure homogenizing apparatus, a millstone friction machine such as a grinder or a mill, a single-screw kneader, a multi-screw kneader, a kneader refiner, or a jet mill. Note that the defibration is preferably performed using a refiner or a jet mill.

The defibration of the raw material pulp is preferably performed such that obtained fine fibrous cellulose has the following desired values or are evaluated as described below for an average fiber diameter, an average fiber length, a water retention degree, a degree of crystallinity, a coefficient of variation in a fiber diameter distribution, a peak value of a pseudo particle size distribution, a pulp viscosity, and a B-type viscosity of a dispersion (slurry).

The fine fibrous cellulose has an average fiber diameter (average fiber width. average of diameters of single fibers) of preferably 1 to 500 nm, more preferably 3 to 500 nm, particularly preferably 10 to 100 nm. When the average fiber diameter of the fine fibrous cellulose is less than 1 nm, the viscosity of the fine fibrous cellulose as a slurry is high, and it is difficult to manufacture a cellulose powder by drying the fine fibrous cellulose. In addition, depending on a manufacturing method, the cellulose may be dissolved, and there is a possibility that a fiber shape cannot be maintained.

On the other hand, when the average fiber diameter of the fine fibrous cellulose is more than 500 nm, the average particle size of the cellulose powder is large, and the cellulose powder has a more distorted shape such as a rod shape or a flat shape, and there is a possibility that a solid powder cosmetic containing the cellulose powder is rough or easily imparts a feeling of a foreign substance.

The average fiber diameter of the fine fibrous cellulose can be adjusted by, for example, selection of a raw material pulp, a pretreatment, or defibration.

A method for measuring the average fiber diameter of the fine fibrous cellulose is as follows.

First, 100 ml of an aqueous dispersion (slurry) of the fine fibrous cellulose having a solid concentration of 0.01 to 0.1% by mass is filtered through a Teflon (registered trademark) membrane filter, and solvent substitution is performed once with 100 ml of ethanol and three times with 20 ml of t-butanol. Next, the resulting product is freeze-dried and coated with osmium to obtain a sample. This sample is observed with an electron microscope SEM image at any magnification of 3,000 to 30,000 depending on the width of a fiber constituting the sample. Specifically, two diagonals are drawn on the observation image, and three straight lines passing an intersection of the diagonals are arbitrarily drawn. Furthermore, the widths of 100 fibers in total intersecting with the three straight lines are visually measured. Then, a median diameter of the measured values is taken as an average fiber diameter.

The average fiber length (average of lengths of single fibers) of the fine fibrous cellulose is preferably 0.3 to 200 µm, more preferably 0.4 to 200 µm, and particularly preferably 0.5 to 200 µm. When the average fiber length of the fine fibrous cellulose is less than 0.3 µm, it is difficult to manufacture a cellulose powder having a desired average particle size due to poor aggregation of fibers, and there is a possibility that a cellulose powder having a small particle size or many aggregated bundle-like particles are manufactured.

On the other hand, when the average fiber length of the fine fibrous cellulose is more than 200 µm, a powder containing various shapes of particles is likely to be formed, which causes unevenness in particle shape and unevenness in particle size.

The average fiber length of the fine fibrous cellulose can be adjusted by, for example, selection of a raw material pulp, a pretreatment, or defibration.

In a method for measuring the average fiber length of the fine fibrous cellulose, similarly to the case of the average fiber diameter, the length of each fiber is visually measured. A median diameter of the measured values is taken as an average fiber length.

The fine fibrous cellulose has an aspect ratio of preferably 50 to 200,000, more preferably 50 to 10,000. When the aspect ratio is less than 50, it is difficult to obtain a cellulose powder having a desired average particle size. On the other hand, when the aspect ratio is more than 200,000, the cellulose powder has a small circularity, and smoothness of the solid powder cosmetic may be impaired.

The degree of crystallinity of the fine fibrous cellulose is preferably 50 or more, more preferably 60 or more, and particularly preferably 70 or more, and the degree of crystallinity of the fine fibrous cellulose is preferably 100 or less, more preferably 95 or less, and particularly preferably 90 or less. When the degree of crystallinity is less than 50, the strength of the cellulose powder is low, and a solid powder cosmetic having excellent impact resistance cannot be obtained in some cases.

The water retention degree of the fine fibrous cellulose is preferably 500% or less, and more preferably 300% to 480% when a chemical modification treatment is not performed. When the water retention degree of the fine fibrous cellulose is less than 300%, defibration is nor performed up to fine fibrous cellulose having a desired size, and as a result, particles having a desired shape and size cannot be obtained in some cases.

On the other hand, when the water retention degree of the fine fibrous cellulose is more than 500%, water retention of the fine fibrous cellulose itself increases, and it takes much time to dry the fine fibrous cellulose.

The water retention degree of the fine fibrous cellulose can be adjusted by, for example, selection of a raw material pulp, a pretreatment, or defibration.

The water retention degree of the fine fibrous cellulose is a value measured in accordance with JAPAN TAPPI No. 26 (2000).

The fine fibrous cellulose has a pulp viscosity of preferably 1.0 to 7.0 mPa•s**,** more preferably 1.5 to 6.5 mPa•s**,** particularly preferably 2.0 to 6.0 mPa•s. The fine fibrous cellulose used for the cellulose powder has a polymerization degree of preferably 150 to 1250, more preferably 250 to 1150. The pulp viscosity is the viscosity of a solution obtained by dissolving cellulose in a copper ethylenediamine solution. The larger the pulp viscosity, the higher the polymerization degree of cellulose. The pulp viscosity is related to the strength and rigidity of a cellulose fiber. When the polymerization degree is too high, a hard cellulose powder is obtained, and even when an additive is mixed, a rough solid powder cosmetic is obtained. On the other hand, when the polymerization degree is too low, the strength of the fiber itself is also lost, a plastic force required for the cellulose powder used in the present invention is excessive, and it is difficult to control moldability of a cosmetic itself. The polymerization degree of the fine fibrous cellulose can be adjusted by, for example, selection of a raw material pulp, a pretreatment, or defibration. When the pulp viscosity is within the above-described range, the particles have good plasticity, and a solid powder cosmetic having impact resistance and smoothness is obtained.

The fine fibrous cellulose obtained by the defibration can be dispersed in an aqueous medium to be a dispersion (slurry), if necessary. The entire aqueous medium is particularly preferably water (aqueous solution). Note that the aqueous medium may be another liquid that is partially compatible with water. As the other liquid, for example, a lower alcohol having 3 or less carbon atoms can be used.

The B-type viscosity of the dispersion of the fine fibrous cellulose (slurry, 2.0% by mass based on solid content) is preferably 400 mPa•s to 100,000 mPa•s**,** and more preferably 500 to 50,000 mPa•s. When the B-type viscosity of the dispersion is within the above-described range, the slurry can be supplied to a drying apparatus without being clogged in a drying step.

The B-type viscosity of the dispersion of the fine fibrous cellulose is a value measured in accordance with JIS-Z8803 (2011) "Method for measuring viscosity of liquid". The B-type viscosity is a resistance torque when a dispersion is stirred, and a higher B-type viscosity means that more energy is required for stirring.

Cellulose fibers can be defibrated by the following defibrating apparatus or method. The defibration can be performed, for example, by using one or more means selected from a homogenizer such as a high-pressure homogenizer or a high-pressure homogenizing apparatus, a stone mill type friction machine such as a grinder or a grinding machine, a refiner such as a conical refiner or a disc refiner, and various bacteria. Note that the cellulose fibers are preferably defibrated using an apparatus or method for making the cellulose fibers finer by a water flow, particularly by a high-pressure water flow. According to this apparatus or method, dimensional uniformity and dispersion uniformity of fine fibrous cellulose to be obtained are very high. In contrast, for example, when a grinder that performs grinding between rotating grindstones is used, it is difficult to make the cellulose fibers finer uniformly, and a fiber mass that cannot be defibrated may partly remain in some cases.

Examples of a grinder used for defibrating the cellulose fibers include Masscolloider manufactured by Masuko Sangyo Co., Ltd. Examples of an apparatus for making the cellulose fibers finer by a high-pressure water flow include Starburst (registered trademark) manufactured by Sugino Machine Co., Ltd. and Nanovater (registered trademark) manufactured by Yoshida Machine Industry Co., Ltd. Examples of a high-speed rotary homogenizer used for defibrating the cellulose fibers include CLEARMIX-11S manufactured by M Technique Co., Ltd.

The solid powder cosmetic of the present embodiment contains a cellulose powder, but may contain a powder Y other than the cellulose powder. In the present specification, a concept including the cellulose powder and the powder Y other than the cellulose powder may be simply referred to as "powder", "powder containing a cellulose powder", or the like.

### (Cellulose powder)

The cellulose powder of the present embodiment is an aggregate formed by drying the fine fibrous cellulose. However, when viewed microscopically, the cellulose powder of the present embodiment includes a cellulose powder formed by drying a single fiber of the fine fibrous cellulose as it is (for example, a cellulose powder formed by entanglement of one yarn in the yarn or a cellulose powder formed by drying up a single fiber of the fine fibrous cellulose), and a cellulose powder formed by aggregation of a plurality of fibers of the fine fibrous cellulose during drying into an aggregate. The fine fibrous cellulose is manufactured from a raw material pulp, and when dried, fibers of the fine fibrous cellulose are wrinkled and shrunk. Therefore, a cellulose powder to be formed has a shape that is difficult to express but has irregularities. For example, the cellulose powder has a shape in which dried up fibers of the fine fibrous cellulose are aggregated, the shape of Kompeito, or a shape formed by crumpling and rolling one or more sheets of Japanese writing paper or the like. The cellulose powder has a white color, a light yellow color, a cream color, a light orange color, or a mixed color of these colors. Particularly, a white or light yellow cellulose powder is not conspicuous as one composition of a solid powder cosmetic, and is preferable.

The cellulose powder of the present embodiment contains the fine fibrous cellulose in an amount of preferably 50% by mass or more, more preferably 60% by mass or more, still more preferably 70% by mass or more, and an upper limit thereof may be 100% by mass. When the mass percentage of the fine fibrous cellulose in the cellulose powder is less than 50% by mass, there is a possibility that the cellulose powder cannot have a desired average particle size or a desired aspect ratio.

In addition, since the cellulose powder has irregularities formed on a surface thereof by fine wrinkles formed on the surface, light emitted from the outside hits the irregularities and is irregularly reflected at various angles. Therefore, the surface of the cellulose powder lacks glossiness, and a solid powder cosmetic containing the cellulose powder has suppressed shininess.

Furthermore, since the cellulose powder of the present embodiment is manufactured by, for example, drying, a plurality of dried up and wrinkled fibers of the fine fibrous cellulose may be entangled to form irregularities on a surface thereof. There are various ways of drying up the fine fibrous cellulose, and the cellulose powder to be formed is formed of particles having a shape formed by a single fiber of the dried fine fibrous cellulose or a shape formed by aggregation of a plurality of fibers of the dried fine fibrous cellulose.

The cellulose powder contained in the solid powder cosmetic of the present embodiment has an average particle size of preferably 1 to 25 µm, more preferably 2 to 24 µm, still more preferably 3 to 23 µm, and an aspect ratio of preferably 0.6 to 0.90, more preferably 0.6 to 0.85. When the average particle size and aspect ratio of the cellulose powder are within the above-described ranges, the solid powder cosmetic has impact resistance and smoothness. In order to achieve both impact resistance and smoothness, it is desirable that both the values of the average particle size and the aspect ratio are within the above-described ranges, and it is not preferable that either one is out of the above-described range. For example, even when the average particle size of the cellulose powder is within the above-described range, impact resistance of the solid powder cosmetic may be impaired when the aspect ratio exceeds the above-described range. Even when the aspect ratio of the cellulose powder is within the above-described range, when the average particle size exceeds the above-described range, the smoothness of the solid powder cosmetic may be impaired. Note that the aspect ratio of the cellulose powder is a value obtained by dividing a minor axis diameter of the cellulose powder by a major axis diameter of the cellulose powder.

The cellulose powder has a moisture content of preferably 1 to 15%, more preferably 3 to 10% in an atmosphere having a humidity of 50%. When the moisture content of the cellulose powder is within the above-described range, the impact resistance of the solid powder cosmetic is further improved. The moisture content of the cellulose powder can be adjusted by the concentration of the fine fibrous cellulose in a fine fibrous cellulose dispersion as a raw material of the cellulose powder, a drying time, the average fiber width of the fine fibrous cellulose, and the like. However, according to a drying method described later, it may be difficult to adjust the moisture content of the cellulose powder to be smaller than the lower limit of the above-described range. When the moisture content of the cellulose powder exceeds the above-described range, the solid powder cosmetic may change in quality after long-term use.

The cellulose powder has a bulk specific gravity of 0.5 g/cm³ or less, preferably 0.1 to 0.5 g/cm³, more preferably 0.1 to 0.4 g/cm³. When the bulk specific gravity of the cellulose powder exceeds 0.5 g/cm³, an adhesion feeling of the solid powder cosmetic (the adhesion feeling refers to a feeling that the solid powder cosmetic sufficiently adheres to an application target such as the skin.) is poor, a moist feeling is hardly felt, and it is difficult for the solid powder cosmetic to be carried by a puff.

The cellulose powder has a circularity of preferably 0.5 to 0.9, more preferably 0.6 to 0.85. When the circularity of the cellulose powder is within the above-described range, a solid powder cosmetic having excellent impact resistance and smoothness is obtained. When the circularity is less than 0.5, the smoothness may decrease, and when the circularity exceeds 0.9, the impact resistance may decrease.

The aspect ratio and the circularity can be measured using Morphologi 4 manufactured by Malvern Panalytical. The aspect ratio and the circularity are numerical values obtained by collecting 3 to 5 mm³ of powder as a sample in a sample cartridge, dispersing the powder on a glass plate using a dispersing unit, and then analyzing the powder at a particle count number of 20,000 by a static image analysis method.

The cellulose powder has compressive strength: 10% strength of preferably 5 MPa or less, more preferably 0.5 to 5 MPa. When the compressive strength of the cellulose powder exceeds 5 MPa, hardness and roughness are felt, and the cellulose powder lacks smoothness.

The cellulose powder has a specific surface area of preferably 1.0 m²/g or more, more preferably 1.0 to 10 m²/g, still more preferably 1.5 to 8 m²/g. When the specific surface area is less than 1.0 m²/g, the cellulose powder has a shape with less irregularities on a surface thereof. Therefore, the solid powder cosmetic has poor adhesiveness, and dryness and powdery texture are conspicuous.

### (Additive)

In order to impart an effect of improving dispersibility of the cellulose powder manufactured through the above-described drying process in the composition of the solid powder cosmetic, an effect of preventing skin dryness, and an effect of imparting moisture (emollient effect), an additive may be added to the cellulose powder. This is because, when the cellulose powder is left after manufacture, particles of the cellulose powder may be aggregated with each other. One of causes for this is considered to be polarity of cellulose molecules constituting the cellulose powder. Therefore, the cellulose powder may be unevenly distributed without being entirely dispersed in the solid powder cosmetic. Therefore, by inclusion of the additive in the cellulose powder, the polarity of the cellulose molecules in the cellulose powder is masked, particles of the cellulose powder are less likely to be aggregated with each other, and the cellulose powder is easily dispersed in the entire solid powder cosmetic. The additive in the present invention may be any additive as long as it can mask the polarity of cellulose molecules or physically prevent strong aggregation of cellulose fibers during drying. The additive is preferably a hydrophilic material considering that the additive is mixed with a dispersion of cellulose fibers in advance, and more preferably a hydrophilic material having an OH group (hydroxy group), a CO group (carbonyl group), or a COOH group (carboxyl group). Since the cellulose powder of the present embodiment is hydrophilic, by inclusion of the additive which is a hydrophilic material, the moisture content of the cellulose powder is maintained within a desired range, and the solid powder cosmetic has excellent impact resistance.

As the additive, one or more selected from the group consisting of a polyhydric alcohol, a polysaccharide, a water-soluble polymer, and a surfactant can be used. The content percentage of the additive in the cellulose powder is preferably 1 to 49% by mass, and more preferably 2 to 48% by mass. When the content percentage is too high, the cellulose powder is sticky or loses its lightweight feeling, and a feeling of adhering to the skin deteriorates. On the other hand, when the content percentage is too low, there is a possibility that the above dispersion effect and emollient effect are not obtained.

Examples of the polyhydric alcohol as the additive include a polyhydric alcohol having 2 to 6 carbon atoms and 2 or 3 oxygen atoms. Specifically, glycerin, diglycerin, propylene glycol, 1,3-butylene glycol, 1,2-pentanediol, dipropylene glycol, 1,2-hexanediol, heptanediol, ethylene glycol, diethylene glycol, 1,3-propanediol, 3-methyl-1,3-butanediol, and the like can be used, but are not limited thereto. In particular, glycerin is preferable from a viewpoint of a thickening property and dispersibility of composite particles.

Examples of the polysaccharide include quince seed, veegum, xanthan gum, and a hyaluronic acid salt, but are not limited thereto. In particular, a hyaluronate or the like is preferable from a viewpoint of a thickening property and dispersibility of the cellulose powder.

Examples of the water-soluble polymer include polyvinyl alcohol, polyvinyl pyrrolidone, a carboxyvinyl polymer, polyethylene glycol, a homopolymer or copolymer containing a monomer having a phosphorylcholine group as a monomer unit, a homopolymer or copolymer containing a monomer having a sugar residue as a monomer unit, and a homopolymer or copolymer containing a monomer having an amino acid residue as a monomer unit. Specific examples thereof include a copolymer formed of an alkyl (meth)acrylate and polymethacryloyloxyethyl phosphorylcholine, a copolymer formed of an alkyl (meth)acrylate and methacryloyloxyethyl glucoside, and a copolymer formed of an alkyl (meth)acrylate and methacryloyl-L-lysine, but are not limited thereto. In particular, polyvinylpyrrolidone is preferable from a viewpoint of a thickening property and dispersibility of the cellulose powder.

Examples of the surfactant include an anionic surfactant, a cationic surfactant, an amphoteric surfactant, and a nonionic surfactant. In particular, a nonionic surfactant is preferable in order to eliminate an influence of ionic bonding. On the other hand, when a cationic surfactant is added to anionic cellulose fibers, the cellulose fibers are excessively aggregated during powder manufacture (during drying), and it may be difficult to control the shape of the powder.

Examples of the nonionic surfactant include a polyoxyalkylene alkyl ether, a glycerin alkyl ether, a glycerin fatty acid ester, a polyglycerin fatty acid ester, a sorbitan fatty acid ester, alkylene glycol adducts thereof, a polyalkylene glycol fatty acid ester, a polyglycerin-modified silicone, and a polyether-modified silicone. Examples of the anionic surfactant include an alkyl phosphate, a polyoxyalkylene alkyl ether phosphate, a sulfonate, an alkyl sulfate, and a polyaspartate. Examples of the cationic surfactant include an alkylamine salt and an alkyltrimethylammonium salt. Examples of the amphoteric surfactant include hydrogenated lecithin, a carbobetaine amphoteric surfactant, a sulfobetaine amphoteric surfactant, and an amino acid amphoteric surfactant.

### (Inorganic fine particles)

The cellulose powder may contain inorganic fine particles. The inorganic fine particles can impart various functions to the cellulose powder. For example, metal-based inorganic fine particles have a function of diffusing and reflecting incident light, and by inclusion of the inorganic fine particles in the cellulose powder, an effect of suppressing irradiation with ultraviolet rays and an effect of suppressing shininess of the skin of an application portion can be expected.

An upper limit of the content percentage of the inorganic fine particles in the cellulose powder is preferably 49% by mass, and the content percentage is preferably 45% by mass or less. A lower limit of the content percentage of the inorganic fine particles is preferably 0% by mass, and the content percentage is preferably 5% by mass or more. When the content percentage exceeds 49% by mass, the specific gravity of the cellulose powder is large due to inclusion of the inorganic fine particles. Therefore, dispersibility in the solid powder cosmetic may be impaired. On the other hand, when the content percentage is 5% by mass or more, the effect of diffusing and reflecting incident light is sufficiently exhibited.

An upper limit of the primary particle size of the inorganic fine particles is preferably 10 µm, and the primary particle size is preferably 5 µm or less, and more preferably 1 µm or less. When the primary particle size of the inorganic fine particles is more than 10 µm, the inorganic fine particles are less likely to be carried by the fine fibrous cellulose. A lower limit of the primary particle size of the inorganic fine particles is not particularly limited, but is preferably 1 nm, and the primary particle size is preferably 2 nm or more, and more preferably 3 nm or more. In a case where the primary particle size of the inorganic fine particles is 1 nm or more, when the inorganic fine particles are mixed with a raw material slurry for manufacturing the cellulose powder, the inorganic fine particles are likely to be dispersed in and cling to the fine fibrous cellulose.

A method for measuring the primary particle size of the inorganic fine particles can be performed by electron microscope observation, and an average value of the obtained particle sizes is taken as a measured value.

The inorganic fine particles can of course be used as they are, but when the inorganic fine particles are subjected to a hydrophilic treatment, the inorganic fine particles are easily acclimated to a raw material slurry for manufacturing the cellulose powder, which is preferable. A surface treatment agent used for the hydrophilic treatment has an effect of suppressing surface activity of the inorganic fine particles, improving dispersibility of the inorganic fine particles, and improving transparency and creakiness. The surface treatment agent for the inorganic fine particles is not particularly limited as long as it is a treatment agent capable of being dispersed in the raw material slurry, but a surface treatment agent containing anhydrous silicic acid or hydrous silicic acid is preferable.

The inorganic fine particles are not particularly limited, and known inorganic fine particles can be used. Examples thereof include barium titanate, lead zirconate titanate, silicon carbide, silicon nitride, aluminum nitride, alumina, zirconia, zircon, titanium oxide, zinc oxide, iron oxide, and cerium oxide. When the effect of suppressing irradiation with ultraviolet rays or the effect of suppressing shininess of the skin is improved, for example, one or a combination of two or more selected from the group consisting of titanium oxide, zinc oxide, iron oxide, and cerium oxide can be used. In particular, when the inorganic fine particles are made of titanium oxide, the effect of suppressing irradiation with ultraviolet rays is improved, which is preferable.

The shapes of the inorganic fine particles that can be contained in the cellulose powder are not particularly limited, but examples thereof include a spherical shape, a rod shape, a needle shape, a spindle shape, a plate shape, and a polygonal shape.

The inorganic fine particles may be attached to a surface of the fine fibrous cellulose in the cellulose powder, or may be encapsulated in the fine fibrous cellulose. When the inorganic fine particles are encapsulated in the fine fibrous cellulose, the inorganic fine particles can be carried not only on a surface of the cellulose powder but also inside the cellulose powder, and even when the cellulose powder is irradiated with incident light from various angles, the cellulose powder diffuses and reflects the incident light, which is preferable. Here, the encapsulation can be said to be a state in which a part of a surface of the inorganic fine particles is covered with the fine fibrous cellulose, or a state in which the inorganic fine particles are covered with the fine fibrous cellulose and cannot be observed from the outside when the cellulose powder is observed from the outside.

The inorganic fine particles can be added to the fine fibrous cellulose dispersion before being dried, and it is preferable to mix the inorganic fine particles and the fine fibrous cellulose so as to be uniform.

The solid powder cosmetic of the present embodiment contains the cellulose powder in an amount of preferably 1 to 99% by mass, more preferably 3 to 60% by mass, still more preferably 5 to 40% by mass. When the content of the cellulose powder is less than 1% by mass, the solid powder cosmetic has poor impact resistance.

### (Manufacture of cellulose powder)

The cellulose powder can be manufactured by drying the fine fibrous cellulose in a drying step. Specifically, the cellulose powder can be manufactured by a method for freeze-drying the fine fibrous cellulose as a raw material, a method for drying the fine fibrous cellulose under reduced pressure, a method for heat-drying the fine fibrous cellulose (for example, a drying method by hot drying), or a method for spray-drying the fine fibrous cellulose. In addition, the cellulose powder can be manufactured by a method for spray-freezing and drying the fine fibrous cellulose under reduced pressure, which is a drying method for the cellulose powder according to the present embodiment. In particular, a cellulose powder manufactured by a heat-drying method or a spray-drying method is suitable as the composition of the solid powder cosmetic of the present embodiment in terms of an average particle size and an aspect ratio.

According to a method for manufacturing the cellulose powder by a drum-drying method among the heat-drying methods, even from fine fibrous cellulose having a relatively high concentration or poor fluidity, a dry body in which particles are hardly aggregated and easily dispersed can be obtained. The manufacture of the cellulose powder by the drum-drying method is performed as follows as an example.

The fine fibrous cellulose can be supplied to, for example, a drum dryer that performs a drying treatment in a slurry (aqueous dispersion) state, and the content (% by absolute dry mass) of the fine fibrous cellulose in this case is 1% by mass or more, preferably 1.5% by mass or more, and more preferably 2.0% by mass or more. The content is 10% by mass or less, preferably 7% by mass or less, and more preferably 5% by mass or less. When the content exceeds 10% by mass, a viscosity of the slurry is too high and a handling property is poor. On the other hand, when the content is less than 1% by mass, much energy and time are consumed to remove moisture, which is not economical.

The drum dryer used in the drying treatment by the drum-drying method may be a known drum dryer. For example, "John Millder JM-T type" manufactured by Johnson Boiler Co., Ltd. can be used. As the drum dryer, an inner-roll-type drum dryer can be suitably used. The inner-roll-type drum dryer performs a gentle drying treatment to provide a dry body having a relatively small specific surface area. The drying treatment can be performed under normal pressure.

Regarding operating conditions of the drum dryer, a surface temperature of an inner surface of a drum is 80 to 200°C, and preferably 90 to 190°C. At this surface temperature, a dry body having a strong cohesive force can be obtained. When the surface temperature exceeds 200°C, some of fibers of the fine fibrous cellulose may be thermally modified. On the other hand, when the surface temperature is lower than 80°C, not only it takes a lot of time to remove moisture but also particles having a very large amount of moisture are obtained. In addition, a rotation speed of the drum dryer can be, for example, 0.5 rpm or more and 2 rpm or less although it depends on the inner diameter of the drum and the amount of the slurry to be put. Time for drying with the drum dryer depends on the amount of the slurry to be put, but if the time is 1 second to 60 seconds, drying is sufficiently performed, and even if drying is performed for a longer time, the water content of a dry body does not decrease any more.

As an apparatus used for manufacturing the cellulose powder by a spray-drying method, a spray-drying apparatus ("P-260" manufactured by PRECi CO,. LTD.) can be exemplified. The cellulose powder can be manufactured by supplying a slurry of fine fibrous cellulose to the spray-drying apparatus. As the spray-drying apparatus, for example, a two-fluid nozzle system including two 90-type nozzles can be used. As conditions for spray-drying, for example, a supply flow rate of a slurry of the fine fibrous cellulose can be 20 kg/h, an inlet temperature of dry air can be 200°C, an outlet temperature thereof can be 100°C, and a spray air pressure can be 0.6 MPa, but the conditions are not limited thereto.

When a cellulose powder containing the above-described additive is manufactured, preferably, the additive is mixed with a slurry of the fine fibrous cellulose as a manufacturing raw material to obtain a mixture, and the mixture is supplied to a drying device (for example, a drum dryer or a spray drying device) used in a method by heat-drying or a method by spray-drying.

### (Oil component)

The solid powder cosmetic of the present embodiment can contain an oil component. By inclusion of an oil component in the solid powder cosmetic, moldability is improved and impact resistance is excellent. The solid powder cosmetic contains the cellulose powder, and therefore can obtain favorable impact resistance even with an oil component amount smaller than that of a conventional cosmetic.

As the oil component, for example, any oil such as animal oil, vegetable oil, or synthetic oil can be used regardless of an origin of oil, and any oil such as solid oil, semi-solid oil, liquid oil, or volatile oil can be used regardless of properties of oil.

As the oil component, for example, one or a combination of two or more selected from hydrocarbons, fats and oils, waxes, hardened oils, ester oils, fatty acids, silicone oils, fluorine-based oils, lanolin derivatives, and oil-soluble ultraviolet absorbers can be used.

More specifically, for example, one or a combination of two or more selected from hydrocarbons such as liquid paraffin, squalane, petrolatum, paraffin wax, ceresin wax, microcrystalline wax, Japan wax, and montan wax; oils and fats such as olive oil, castor oil, jojoba oil, mink oil, and macadamia nut oil; waxes such as beeswax, lanolin, carnauba wax, candelilla wax, and spermaceti; esters such as isostearyl isostearate, cetyl isooctanate, isopropyl myristate, isopropyl palmitate, octyldodecyl myristate, glyceryl trioctanoate, glyceryl tribehenate, pentaerythritol rosinate, diisostearyl malate, dilinoleyl dimer dilinoleate, and neopentyl glycol dioctanoate; silicones such as dimethylpolysiloxane having a low polymerization degree, dimethylpolysiloxane having a high polymerization degree, methylphenylpolysiloxane, a silicone gel containing a crosslinked silicone such as a (dimethicone/vinyl dimethicone) cross polymer and a solvent, and fluorine-modified silicone; fluorine-based oil agents such as perfluoropolyether, perfluorodecane, and perfluorooctane; lanolin derivatives such as lanolin, lanolin acetate, lanolin fatty acid isopropyl, and lanolin alcohol; lipophilic surfactants having an HLB value of 8 or less, such as sorbitan isostearate, sorbitan sesquiisostearate, polyglyceryl-2 isostearate, and polyglyceryl-2 diisostearate; and oil-soluble ultraviolet absorbers such as ethylhexyl methoxycinnamate, dimethyl PABA ethylhexyl, diethylaminohydroxybenzoylhexyl benzoate, ethylhexyltriazone, and bisethylhexyloxyphenol methoxyphenyltriazine can be used. Among these components, when one or a combination of two or more selected from high-viscosity ester oils having a viscosity of 1,000 to 50,000 mPa•s at 25°C, such as diisostearyl malate and dilinoleyl dimer dilinoleate, and lipophilic surfactants such as sorbitan sesquiisostearate and polyglyceryl-2 diisostearate is used, moldability and moist touch are further improved.

The content of the oil component in the solid powder cosmetic of the present embodiment is preferably 0.1 to 40% by mass, more preferably 0.1 to 25% by mass, and particularly preferably 1 to 15% by mass. When the content of the oil component is less than 0.1% by mass, moldability may be deteriorated. On the other hand, when the content of the oil component is more than 40% by mass, the cosmetic is poorly carried by a cosmetic puff such as a sponge, and it is difficult to spread the cosmetic over the skin. Since the solid powder cosmetic of the present embodiment contains the cellulose powder, the solid powder cosmetic can maintain moldability with an oil component amount smaller than a normal oil component amount, and therefore can be a cosmetic that is favorably carried by a puff and favorably spread over the skin.

### (Other powder Y)

The powder Y other than the cellulose powder can be blended in the solid powder cosmetic of the present embodiment. When the powder Y is contained, a sticky feeling of a cosmetic film can be further suppressed, and when the solid powder cosmetic of the present embodiment is used as a base makeup cosmetic, a covering force and a finish feeling can be made desired. The powder Y other than the cellulose powder has an average particle size of 0.01 to 200 µm.

The powder Y other than the cellulose powder is not limited by, for example, a shape such as a spherical shape, a plate shape, a spindle shape, or a needle shape, a particle size, and a particle structure such as a porous particle or a non-porous particle. The powder Y other than the cellulose powder can be one or a combination of two or more selected from an inorganic coloring pigment, an inorganic extender pigment, an inorganic fine particle powder, a glitter powder, an organic extender pigment, and an organic coloring pigment.

As the powder Y other than the cellulose powder, specifically, for example, one or a combination of two or more selected from an inorganic coloring pigment such as titanium oxide, zinc oxide, zirconium oxide, red iron oxide, yellow iron oxide, black iron oxide, carbon black, chromium hydroxide, iron blue, or ultramarine; an inorganic extender pigment such as mica, sericite, talc, kaolin, synthetic phlogopite, anhydrous silicic acid (silica), magnesium carbonate, calcium carbonate, aluminum hydroxide, alumina, aluminum silicate, magnesium silicate, aluminum magnesium silicate, silicon carbide, barium sulfate, calcium silicate, zeolite, calcined calcium sulfate (calcined gypsum), calcium phosphate, hydroxyapatite, or boron nitride; an inorganic fine particle powder of titanium oxide, zinc oxide, or cerium oxide having an average primary particle size of 100 nm or less; a sparkling powder such as bismuth oxychloride, mica titanium, iron oxide-coated mica, iron oxide-coated mica titanium, organic pigment-coated mica titanium, or aluminum powder; an organic extender pigment such as magnesium stearate, zinc stearate, N-acyllysine, polyurethane, polystyrene, nylon, polymethyl methacrylate, polymethyl silsesquioxane powder, organopolysiloxane elastomer powder, cellulose other than the cellulose powder, crystalline cellulose, polyethylene, crosslinked polymethyl (meth)acrylate, polyester, a copolymer of styrene and acrylic acid, benzoguanamine, tetrafluoroethylene, or cellulose acetate; and an organic coloring pigment including an organic pigment such as red No. 201, red No. 202, red No. 204, red No. 205, red No. 220, red No. 226, red No. 228, red No. 405, orange No. 203, orange No. 204, yellow No. 205, yellow No. 401, or blue No. 404, lakes (zirconium lake, barium lake, and aluminum lake) of a water-soluble dye such as red No. 3, red No. 104, red No. 106, red No. 227, red No. 230, red No. 401, red No. 505, orange No. 205, yellow No. 4, yellow No. 5, yellow No. 202, yellow No. 203, green No. 3, or blue No. 1, and natural dyes and lakes thereof can be used.

The powder Y other than the cellulose powder is preferably subjected to a surface hydrophobizing treatment from a viewpoint of use touch and makeup sustainability. As such a surface hydrophobizing treatment, for example, a known surface hydrophobizing treatment such as a baking treatment with silicone such as methylhydrogenpolysiloxane or a (dimethicone/methicone) copolymer, a fatty acid treatment with stearic acid, a fatty acid metal soap treatment with aluminum stearate or zinc stearate, an acylated amino acid treatment, a lipoamino acid treatment which is a mixing treatment of an acylated amino acid (salt) and a fatty acid (salt), a fluorine treatment with perfluoroalkylphosphate, a silylation treatment with trimethylsilane, or an acidic ester treatment with isostearyl sebacate can be used.

The solid powder cosmetic of the present embodiment preferably contains an inorganic extender pigment subjected to a surface hydrophobizing treatment from a viewpoint of improving makeup sustainability and a use feeling such as adhesion to the skin or ease of spreading. Preferable examples of an inorganic extender pigment constituting the inorganic extender pigment subjected to the surface hydrophobizing treatment include a plate-shaped powder having a volume average particle size of 1 to 50 um. Specific examples of the plate-shaped powder include talc, sericite, mica, synthetic phlogopite, and boron nitride. The surface hydrophobizing treatment in the inorganic extender pigment subjected to the surface hydrophobizing treatment is preferably an acidic ester treatment, an acylated amino acid treatment, or a lipoamino acid treatment because excellent moldability, moist feeling, and makeup sustainability are obtained. Preferable specific examples of the surface hydrophobizing treatment include an isostearyl sebacate treatment which is an acidic ester treatment, an Al stearoyl glutamate treatment which is acylated amino acid treatment, and a palmitoylproline/Mg palmitoyl glutamate/Na palmitoylsarcosine/Al palmitate composite treatment which is lipoamino acid treatment.

Among the above powders Y other than the cellulose powder, in particular, when a spherical inorganic powder or organic powder, for example, spherical silica, polyurethane, polystyrene, nylon, polymethyl methacrylate, a polymethyl silsesquioxane powder, an organopolysiloxane elastomer powder, cellulose other than the above cellulose powder, crystalline cellulose, cellulose acetate, or the like is used, irregularities such as pores and fine wrinkles can be effectively concealed, and therefore a preferable finish is obtained.

The content of a powder containing the cellulose powder (that is, a combination of the cellulose powder and the powder Y other than the cellulose powder) is preferably 60 to 99.9% by mass, more preferably 70 to 99% by mass, and particularly preferably 80 to 95% by mass with respect to the total amount of the solid powder cosmetic. When the content of the powder containing the cellulose powder is less than 60% by mass, the powder is poorly carried by a cosmetic puff such as a sponge, and a formed cosmetic film tends to be sticky.

When the solid powder cosmetic of the present embodiment contains a plate-shaped inorganic extender pigment, the content of the plate-shaped inorganic extender pigment is preferably 1 to 95% by mass, more preferably 5 to 90% by mass, and particularly preferably 10 to 85% by mass with respect to the total amount of the solid powder cosmetic. Here, the plate shape is not particularly limited, but for example, refers to a particle shape having an aspect ratio (length/thickness) of 5 or more, the aspect ratio being calculated by observing a particle using a scanning electron microscope (SEM) and measuring the thickness and length of each cross section. When the content of the plate-shaped inorganic extender pigment is small, adhesiveness (adhesion to the skin) tends to be poor. On the other hand, when the plate-shaped inorganic extender pigment is contained in a large amount, there is a concern that moldability and falling strength are impaired. However, in the solid powder cosmetic of the present embodiment, even when the plate-shaped inorganic extender pigment is blended in an amount of, for example, more than 70% by mass, the cellulose powder improves moldability, and therefore excellent moldability and falling strength are obtained.

When the solid powder cosmetic of the present embodiment contains a spherical inorganic powder and/or organic powder, the content of the spherical inorganic powder and/or organic powder is preferably 1 to 40% by mass, more preferably 3 to 35% by mass, and particularly preferably 5 to 30% by mass with respect to the total amount of the solid powder cosmetic. Here, the spherical shape refers to, for example, a shape having a circularity of 0.8 to 1 obtained by the static image analysis method described above. When the content is excessively small, an effect of concealing irregularities is poor, and when the content is excessively large, moldability is deteriorated.

### (Moisturizing agent)

A moisturizing agent can be blended in the solid powder cosmetic of the present embodiment. As the moisturizing agent, for example, one or a combination of two or more selected from polyhydric alcohols, saccharides, sugar alcohols, amino acids, peptides, and water-soluble polymers can be used. In addition, as the moisturizing agent, for example, glycerin, diglycerin, propylene glycol, 1,3-butylene glycol, 1,2-pentanediol, dipropylene glycol, 1,3-propanediol, 1,2-hexanediol, heptanediol, 1,2-octanediol, ethylhexylglycerin, xylitol, sorbitol, maltitol, chondroitin sulfate, hyaluronic acid, collagen, sodium lactate, dl-pyrrolidone carboxylate, Rosa roxburghii extract, Achillea Millefolium extract, or Melilot extract can be blended.

### (Others)

In the solid powder cosmetic of the present embodiment, in addition to the powder, oil component, and moisturizing agent described above, various components to be blended in an ordinary cosmetic, such as a water-soluble ultraviolet absorber, an antiseptic/antibacterial agent, a fragrance, an antioxidant, a pH adjuster, a chelating agent, a refreshing agent, an anti-inflammatory agent, a skin beautifying component, vitamins, amino acids, a nucleic acid, and an inclusion compound can be blended.

### (Application and the like)

The solid powder cosmetic of the present embodiment is particularly excellent for use as a makeup cosmetic such as a pressed powder (solid white powder), a powder foundation, a foundation usable in both a wet state and a dry state, a cake foundation usable only in a wet state, a powder eye shadow, a cheek rouge, a face color, or an eyebrow ink.

### Examples

Next, results of various test performed will be described. Cellulose powders, a silicone powder, and a silica powder used in Test Examples and Reference Examples were obtained as follows. Note that a product "ELLEX (registered trademark)-S" manufactured by Daio Paper Co., Ltd. was used as fine fibrous cellulose, and special grade glycerin manufactured by FUJIFILM Wako Pure Chemical Corporation was used as glycerin.

### (Cellulose powder A)

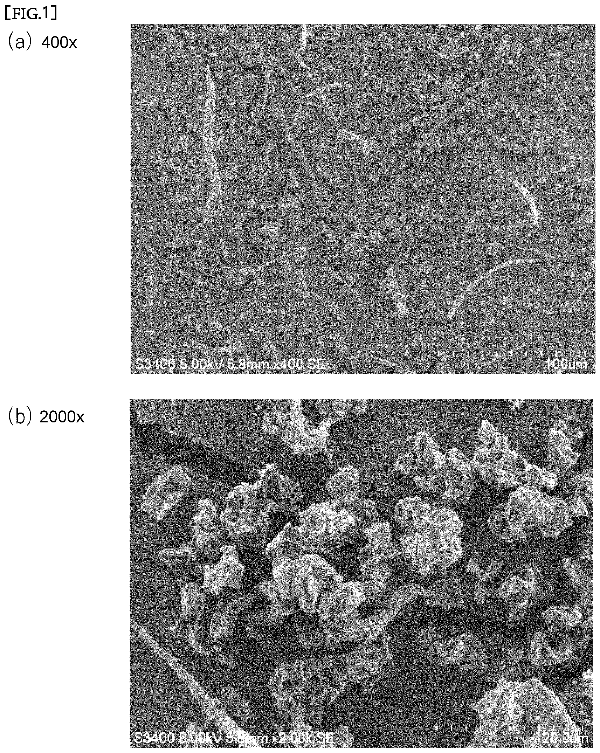

The fine fibrous cellulose and the glycerin were mixed so as to be 70% by mass and 30% by mass on a solid content basis, respectively, to obtain a fine fibrous cellulose aqueous dispersion having a concentration of 3.0%. The aqueous dispersion was spray-dried using a spray dryer ("P-260" manufactured by PRECi CO,. LTD.) to obtain a cellulose powder A. The cellulose powder A had an average particle size of 13.5 µm. An SEM image of the cellulose powder A is illustrated in Fig. 1.

### (Cellulose powder B)

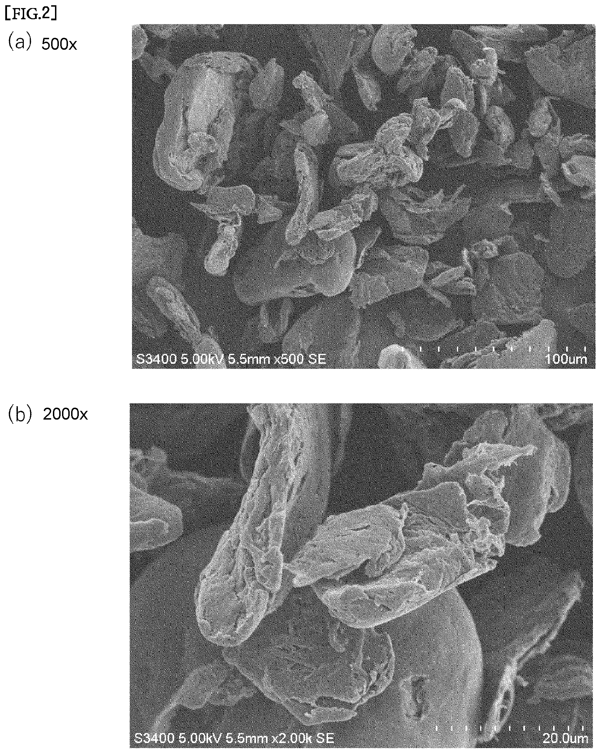

The fine fibrous cellulose and the glycerin were mixed with water so as to be 70% by mass and 30% by mass on a solid content basis, respectively, to obtain a fine fibrous cellulose aqueous dispersion. The aqueous dispersion was dried with a double drum dryer ("John Milder JM-T type" manufactured by Johnson Boiler Co., Ltd.) at a drum rotation number of 3 rpm and a drum surface temperature of 135°C to obtain a dry body, and the dry body was pulverized to obtain a cellulose powder B. An SEM image of the cellulose powder B is illustrated in Fig. 2.

### (Cellulose powder C)

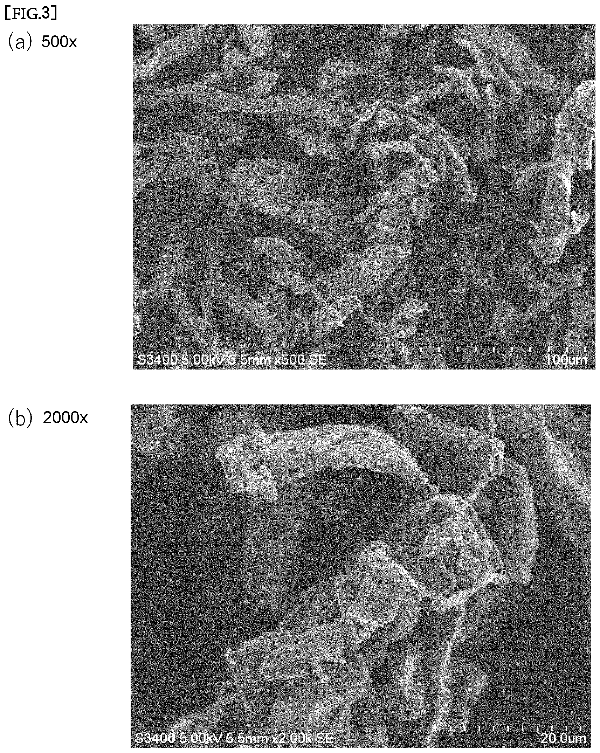

As a cellulose powder C, a product "Wako primary cellulose, powder, passing through 38 µm (400 mesh)" manufactured by FUJIFILM Wako Pure Chemical Corporation was used. Note that a cellulose raw material of the cellulose powder C is not the fine fibrous cellulose used in the present invention. An SEM image of the cellulose powder C is illustrated in Fig. 3.

### (Cellulose powder D)

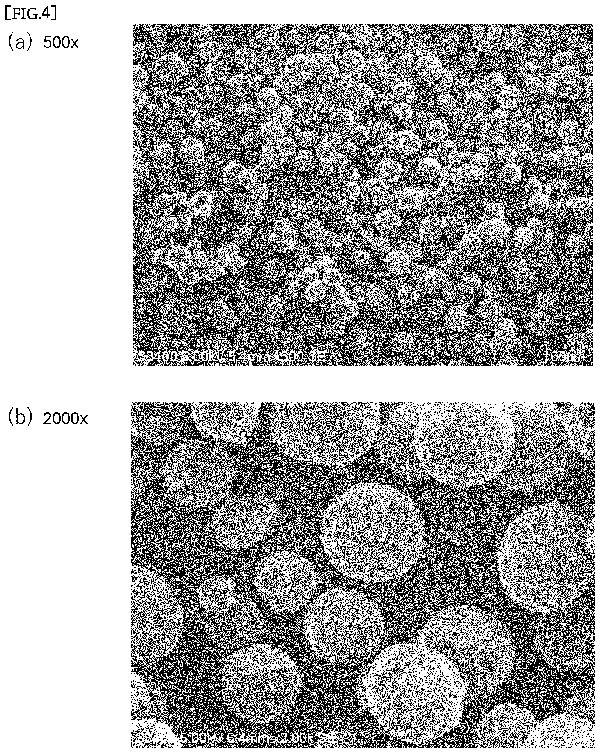

As a cellulose powder D, a product "CELLULOBEADS" manufactured by Daito Kasei Co., Ltd. was used. Note that a cellulose raw material of the cellulose powder D is not the fine fibrous cellulose used in the present invention. An SEM image of the cellulose powder D is illustrated in Fig. 4.

### (Cellulose powder E)

The fine fibrous cellulose was dispersed in water so as to have a concentration of 3.0% on a solid content basis to obtain a fine fibrous cellulose aqueous dispersion. No additive was added. The aqueous dispersion was spray-dried using a spray dryer ("P-260" manufactured by PRECi CO,. LTD.) to obtain a cellulose powder E.

### (Silicone powder)

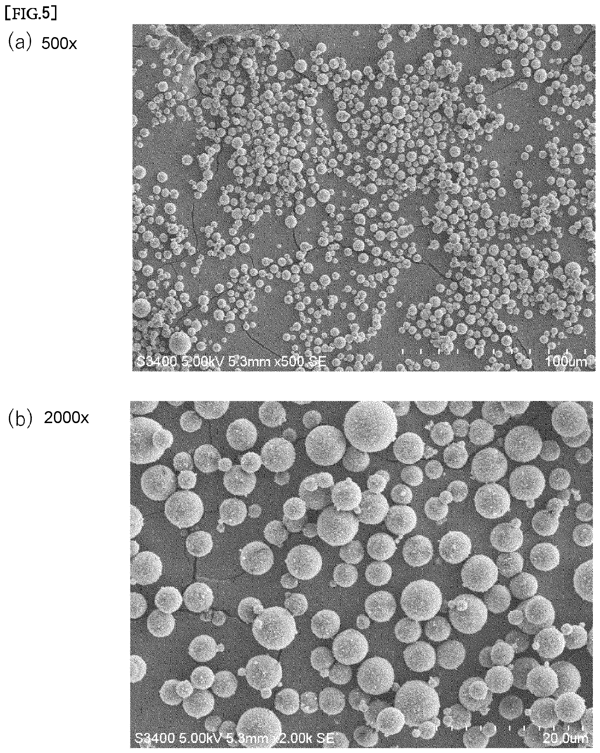

A product "KSP-100" manufactured by Shin-Etsu Chemical Co., Ltd. was used as a silicone powder. An SEM image of the silicone powder is illustrated in Fig. 5.

### (Silica powder)

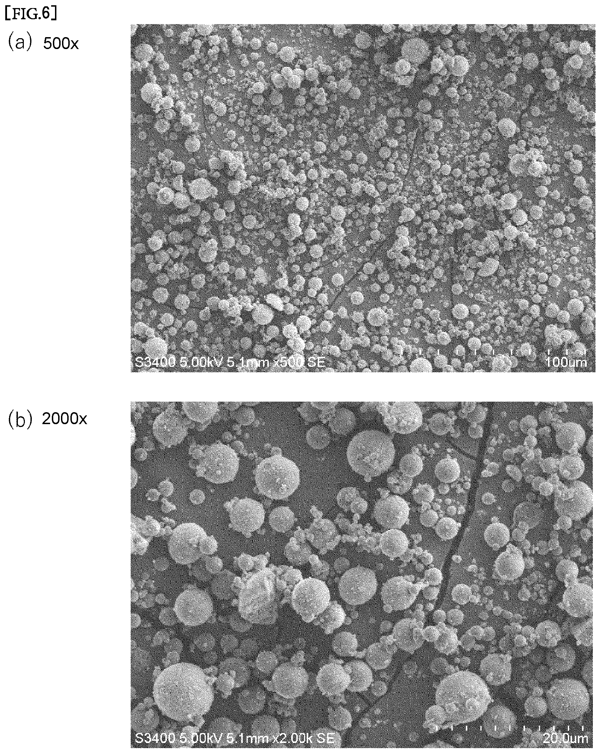

As a silica powder, a product "CHIFFONSIL P-3R" manufactured by JGC Catalysts and Chemicals Ltd. was used. An SEM image of the silica powder is illustrated in Fig. 6.

Physical properties of the obtained powders were measured. Measurement items of the physical properties are as presented in Table 1. The measurement items of the physical properties were measured according to the following procedure.

A bulk specific gravity was measured by a static method in accordance with JIS-K-5101-12-1 (2004).

A moisture content was determined by allowing 5 g of powder to stand for 24 hours by a heat-drying method, and measuring the weight thereof before and after drying. More specifically, the powder that had been allowed to stand for 24 hours or more under a condition of a humidity of 50% (that is, the powder before drying) was dried at a temperature of 105°C for 24 hours to obtain a powder after drying, and the moisture content was calculated from the weight of the powder before drying and the weight of the powder after drying according to the following mathematical formula [Mathematical Formula 1]. (Moisture content (%)) = [((weight of powder before drying) - (weight of powder after drying))/(weight of powder after drying)] ×100

Compressive strength was measured by a micro component strength evaluation apparatus Microautograph "MST-I" manufactured by SHIMADZU CORPORATION with reference to JIS R1639-5 "Fine ceramics-Method for measuring grain characteristics-Part 5: Single grain collapse strength".

A specific surface area (BET multipoint method) was measured with a specific surface area measuring apparatus "TriStar II 3020 N₂ gas" manufactured by Shimadzu Corporation.

A circle equivalent diameter integrated distribution (number basis, integrated 50% diameter), a circle equivalent diameter integrated distribution (volume basis, integrated 50% diameter), an aspect ratio, and a circularity were measured by a product "Morphologi 4" manufactured by Spectris.

A mode diameter, a median diameter (integrated 50% diameter), an average particle size, an integrated 10% diameter, and an integrated 90% diameter were measured by a dry method without splashing off moisture attached to a cellulose powder using a measuring apparatus in accordance with ISO-13320 (2009), specifically, a laser diffraction/scattering type particle size distribution measuring apparatus (particle size distribution) "LA-960V2".

For each of the obtained cellulose powders A to D, a pulp viscosity of cellulose and an average polymerization degree of cellulose were measured. The pulp viscosity is a value measured in accordance with TAPPI T 230. The average polymerization degree of cellulose refers to a viscosity average polymerization degree measured in accordance with JIS-K6726.

**[Table 1]**

| Measurement items of physical properties | Unit | Cellulose powder A | Cellulose powder B | Cellulose powder C | Cellulose powder D | Silicone powder | Silica powder |
|---|---|---|---|---|---|---|---|
| Bulk specific gravity | g/cm³ | 0.25 | 0.23 | 0.26 | 0.72 | 0.33 | 0.71 |
| Moisture content | % | 4.67 | 9.55 | 4.69 | 6.48 | 0.70 | 1.54 |
| Compressive strength | 10% strength, Mpa | 3.82 | 0.85 | 2.75 | 26.37 | 0.82 | 1.17 |
| Specific surface area (BET multipoint method) | m²/g | 2.07 | 0.97 | 1.53 | 0.39 | 2.03 | 0.88 |
| Circle equivalent diameter integrated distribution (number basis, integrated 50% diameter) | µm | 6.45 | 15.00 | 10.50 | 10.80 | 4.64 | 2.70 |
| Circle equivalent diameter integrated distribution (volume basis, integrated 50% diameter) | µm | 17.00 | 49.30 | 34.30 | 12.50 | 5.58 | 15.30 |
| Aspect ratio | | 0.63 | 0.57 | 0.54 | 0.94 | 0.96 | 0.88 |
| Circularity | | 0.73 | 0.64 | 0.67 | 0.98 | 0.99 | 0.96 |
| Mode diameter | µm | 10.8 | 48.3 | 24.4 | 16.3 | - | 8.2 |
| Median diameter (integrated 50% diameter) | µm | 10.9 | 47.6 | 29.9 | 16.1 | - | 8.2 |
| Average particle size | µm | 13.5 | 53.1 | 41.5 | 16.6 | - | 8.8 |
| Integrated 10% diameter | µm | 6.4 | 22.3 | 12.8 | 10.3 | - | 4.7 |
| Integrated 90% diameter | µm | 20.0 | 88.3 | 83.7 | 23.6 | - | 13.7 |
| Pulp viscosity | mPa·s | 2.9 | 2.4 | 2.3 | 2.0 | | |
| Average polymerization degree | | 509 | 421 | 403 | 351 | | |

### (Test Examples and Reference Examples)

The powders obtained as described above were blended at blending ratios presented in Tables 2 and 3. Specifically, for Test Examples 1 and 2 and Reference Examples 1 to 3 in Table 2, a mixture obtained by mixing any one of powders of component Nos. 1 to 5 and component Nos. 6 to 13 was mixed at 10,000 rpm (scale 50) for 10 seconds using a sample mill (product "SK-M10" manufactured by Kyoritsu Rikoh Co., Ltd.) to obtain a mixture as an intermediate product. Subsequently, a mixture obtained by uniformly mixing components of component Nos. 14 to 19 in advance was mixed with the above-described mixture, and the resulting mixture was further mixed with the above-described sample mill for 30 seconds to obtain each of powder compositions for Test Examples 1 and 2 and Reference Examples 1 to 3.

For Test Example 3 and Reference Examples 4 to 7 in Table 3, a mixture obtained by mixing any one of powders of component Nos. 1 to 4 and component Nos. 5 to 13 was mixed at 10,000 rpm (scale 50) for 10 seconds using a sample mill (product "SK-M10" manufactured by Kyoritsu Rikoh Co., Ltd.) to obtain a mixture as an intermediate product. Subsequently, a mixture obtained by uniformly mixing components of component Nos. 14 to 19 (component Nos. 14 to 20 for Reference Example 7) in advance was mixed with the above-described mixture, and the resulting mixture was further mixed with the above-described sample mill for 30 seconds to obtain each of powder compositions for Test Example 3 and Reference Examples 4 to 7.

**[Table 2]**

| Component No. | Component name | Test Example 1 | Test Example 2 | Reference Example 1 | Reference Example 2 | Reference Example 3 |
|---|---|---|---|---|---|---|
| 1 | Cellulose powder A | 11.00 | | | | |
| 2 | Cellulose powder B | | 11.00 | | | |
| 3 | Cellulose powder D*1 | | | 11.00 | | |
| 4 | Silicone powder*2 | | | | 11.00 | |
| 5 | Silica powder*3 | | | | | 11.00 |
| 6 | Synthetic phlogopite | 10.00 | 10.00 | 10.00 | 10.00 | 10.00 |
| 7 | Boron nitride | 5.00 | 5.00 | 5.00 | 5.00 | 5.00 |
| 8 | Silicone-coated mica | 5.00 | 5.00 | 5.00 | 5.00 | 5.00 |
| 9 | Acylamino acid-coated talc*4 | 38.69 | 38.69 | 38.69 | 38.69 | 38.69 |
| 10 | Isostearyl sebacate-coated talc*5 | 20.00 | 20.00 | 20.00 | 20.00 | 20.00 |
| 11 | Silicone-coated red iron oxide | 0.10 | 0.10 | 0.10 | 0.10 | 0.10 |
| 12 | Silicone-coated yellow iron oxide | 0.20 | 0.20 | 0.20 | 0.20 | 0.20 |
| 13 | Silicone-coated black iron oxide | 0.01 | 0.01 | 0.01 | 0.01 | 0.01 |
| 14 | 25:75 mixture of (dimethicone/vinyl dimethicone) cross polymer and dimethicone*6 | 0.60 | 0.60 | 0.60 | 0.60 | 0.60 |
| 15 | Dimethicone (100 mm³/s) | 0.40 | 0.40 | 0.40 | 0.40 | 0.40 |
| 16 | Isostearyl isostearate | 5.00 | 5.00 | 5.00 | 5.00 | 5.00 |
| 17 | Sorbitan sesquiisostearate | 0.50 | 0.50 | 0.50 | 0.50 | 0.50 |
| 18 | Diisostearyl malate | 2.00 | 2.00 | 2.00 | 2.00 | 2.00 |
| 19 | Ethylhexylglycerin | 1.50 | 1.50 | 1.50 | 1.50 | 1.50 |
| | Total | 100.00 | 100.00 | 100.00 | 100.00 | 100.00 |

| | | | | | | |
|---|---|---|---|---|---|---|
| *1 trade name: CELLULOBEADS D-10 manufactured by Daito Kasei Co., Ltd. *2 trade name: KSP-100 manufactured by Shin-Etsu Chemical Co., Ltd. *3 trade name: CHIFFONSIL P-3R manufactured by JGC Catalysts and Chemicals Ltd. *4 trade name: NAI-talc JA-46R manufactured by Miyoshi Kasei, Inc. *5 trade name: HS-talc JA-46R manufactured by Miyoshi Kasei, Inc. *6 trade name: KSG-16 manufactured by Shin-Etsu Chemical Co., Ltd. | | | | | | |

**[Table 3]**

| Component No. | Component name | Test Example 3 | Reference Example 4 | Reference Example 5 | Reference Example 6 | Reference Example 7 |
|---|---|---|---|---|---|---|
| 1 | Cellulose powder A | 11.00 | | | | |
| 2 | Cellulose powder C | | 11.00 | | | |
| 3 | Cellulose powder D*1 | | | 11.00 | | |
| 4 | Cellulose powder E*2 | | | | 11.00 | 7.70 |
| 5 | Synthetic phlogopite | 10.00 | 10.00 | 10.00 | 10.00 | 10.00 |
| 6 | Boron nitride | 5.00 | 5.00 | 5.00 | 5.00 | 5.00 |
| 7 | Silicone-coated mica | 5.00 | 5.00 | 5.00 | 5.00 | 5.00 |
| 8 | Acylamino acid-coated talc*3 | | | | | |
| 9 | Silicone-coated talc*4 | 44.64 | 44.64 | 44.64 | 44.64 | 44.64 |
| 10 | Isostearyl sebacate-coated talc*5 | 20.00 | 20.00 | 20.00 | 20.00 | 20.00 |
| 11 | Silicone-coated red iron oxide | 0.10 | 0.10 | 0.10 | 0.10 | 0.10 |
| 12 | Silicone-coated yellow iron oxide | 0.20 | 0.20 | 0.20 | 0.20 | 0.20 |
| 13 | Silicone-coated black iron oxide | 0.01 | 0.01 | 0.01 | 0.01 | 0.01 |
| 14 | 25:75 mixture of (dimethicone/vinyl dimethicone) cross polymer and dimethicone*6 | 0.18 | 0.18 | 0.18 | 0.18 | 0.18 |
| 15 | Dimethicone (100 mm³/s) | 0.12 | 0.12 | 0.12 | 0.12 | 0.12 |
| 16 | Isostearyl isostearate | 1.50 | 1.50 | 1.50 | 1.50 | 1.50 |
| 17 | Sorbitan sesquiisostearate | 0.15 | 0.15 | 0.15 | 0.15 | 0.15 |
| 18 | Diisostearyl malate | 0.60 | 0.60 | 0.60 | 0.60 | 0.60 |
| 19 | Ethylhexylglycerin | 1.50 | 1.50 | 1.50 | 1.50 | 1.50 |
| 20 | Glycerin | | | | | 3.30 |
| | Total | 100.00 | 100.00 | 100.00 | 100.00 | 100.00 |

| | | | | | | |
|---|---|---|---|---|---|---|
| *1 trade name: CELLULOBEADS D-10 manufactured by Daito Kasei Co., Ltd. *2 Cellulose powder E was manufactured by drying only fine fibrous cellulose without being mixed with an additive using a spray dryer, and had an average particle size of 15 µm *3 trade name: NAI-talc JA-46R manufactured by Miyoshi Kasei, Inc. *4 trade name: SA-talc JA-46R manufactured by Miyoshi Kasei, Inc. *5 trade name: HS-talc JA-46R manufactured by Miyoshi Kasei, Inc. *6 trade name: KSG-16 manufactured by Shin-Etsu Chemical Co., Ltd. | | | | | | |

Each of the powder compositions obtained according to the above operation procedure was filled into an aluminum metal dish having a diameter of 54 mmϕ, and press-molded at a press pressure presented in Table 4 using a press machine (semi-automatic press machine "SSPP (hydraulic cylinder diameter: 80 mmϕ)" manufactured by Sanshin Seiki Co., Ltd.) to obtain each of Test Examples 1 to 3 and Reference Examples 1 to 7. Hardness of each of the obtained Test Examples 1 to 3 and Reference Examples 1 to 7 was measured. The hardness was measured under a condition of a load of 1 pound (= 453.59 g) using a product "Orzen hardness tester" manufactured by Ueshima Seisakusho Co., Ltd., which is a constant load type indentation hardness tester. Measurement results of the hardness are presented un Table 4.

**[Table 4]**

| Molding condition | Test Example 1 | Test Example 2 | Reference Example 1 | Reference Example 2 | Reference Example 3 | Test Example 3 | Reference Example 4 | Reference Example 5 | Reference Example 6 | Reference Example 7 |
|---|---|---|---|---|---|---|---|---|---|---|
| Press pressure (Mpa) | 1.8 | 1.8 | 1.8 | 1.8 | 1.8 | 2.6 | 2.2 | 2.6 | 1.8 | 18 |
| Hardness | 27 | 31 | 26 | 47 | 29 | 19 | 26 | 27 | 27 | 30 |

### (Performance evaluation test)

A performance evaluation test was performed on Test Examples and Reference Examples. Test items of the performance evaluation test are falling strength, ease of being carried by a puff, smoothness at the time of application to the skin, a moist feeling at the time of application to the skin, and the degree of adhesion to the skin. Results of the performance evaluation test are presented in Table 5.

**[Table 5]**

| Evaluation item | Test Example 1 | Test Example 2 | Reference Example 1 | Reference Example 2 | Reference Example 3 | Test Example 3 | Reference Example 4 | Reference Example 5 | Reference Example 6 | Reference Example 7 |
|---|---|---|---|---|---|---|---|---|---|---|
| Falling strength | A (>11) | A (>11) | B (10) | D (4) | B (8) | B (8) | C (6) | D (3) | D (4) | B (10) |
| Ease of being carried by puff | A | C | D | A | C | A | B | C | A | C |
| Smoothness at time of application | A | B | A | A | A | A | A | A | B | B |
| Moist feeling at time of application | A | B | C | A | C | B | C | C | B | A |
| Degree of adhesion to skin | A | B | D | A | C | A | B | C | A | C |

A test for falling strength was performed according to the following operation procedure.
(1) From a height of 30 cm above a horizontally disposed concrete block plane, Test Example or Reference Example was caused to freely fall with a planar part of the Test Example or the Reference Example horizontal to be caused to collide with the concrete block plane.
(2) The operation of (1) was repeated until the Test Example or the Reference Example cracked.
(3) A case where the number of times of trial in (1) and (2) before the Test Example or the Reference Example cracked was 11 or more was evaluated as Judgment A, a case where the number of times of trial was 8 or more and 10 or less was evaluated as Judgment B, a case where the number of times of trial was 5 or more and 7 or less was evaluated as Judgment C, and a case where the number of times of trial was 4 or less was evaluated as Judgment D. As for the notation of the falling strength in Table 5, for example, when the Test Example or the Reference Example cracked in the number of times of trial of 10, it was described as "B (10)" or the like.

Tests for ease of being carried by a puff, smoothness at the time of application to the skin, a moist feeling at the time of application to the skin, and the degree of adhesion to the skin were performed as follows. Ten female specialized panels used and evaluated Test Examples and Reference Examples.

Specifically, each of ease of being carried by a puff, smoothness at the time of application to the skin, a moist feeling at the time of application to the skin, and the degree of adhesion to the skin was evaluated with three levels of scores: a score 2 when a sample was felt to be good, a score 1 when a sample was felt not to be good and not to be bad, and a score 0 when a sample was felt to be bad. An average score was obtained from the obtained scores, and when the average score was 1.5 or more, it was evaluated as Judgment A, when the average score was 1.2 or more and less than 1.5, it was evaluated as Judgment B, when the average score was 0.5 or more and less than 1.2, it was evaluated as Judgment C, and when the average score was less than 0.5, it was evaluated as Judgment D.

The results in Table 5 revealed the following. When comparing Test Examples 1 and 2 and Reference Examples 1 to 3 which were about the same in terms of the powder amount (90% by mass) and the total amount (10% by mass) of the oil component and the moisturizing agent, Test Example 1 containing cellulose powder A was excellent in all the items, whereas Reference Example 1 containing a commercially available spherical cellulose and Reference Example 3 containing a commercially available spherical silica were largely inferior in items of ease of being carried by a puff, a moist feeling at the time of application, and the degree of adhesion to the skin. In Reference Example 2 containing a silicone elastomer which is an elastic powder, cosmetic performance such as a moist feeling was equivalent to that of Test Example 1, but falling strength was significantly inferior to that of Test Example 1. Test Example 2 containing cellulose powder B having a large average particle size was excellent in falling strength, but was slightly inferior to Test Example 1 in items of ease of being carried by a puff, smoothness at the time of application, a moist feeling at the time of application, and the degree of adhesion to the skin.

When comparing Test Example 3 and Reference Examples 4 to 7 which were about the same in terms of the powder amount (95.95% by mass) and the total amount (4.05% by mass) of the oil component and the moisturizing agent, Test Example 3 was excellent in all the items even when the total amount of the oil component and the moisturizing agent was small, whereas all of Reference Examples 4 to 6 had insufficient falling strength. On the other hand, Reference Example 4 containing commercially available cellulose powder C had poor falling strength and a slightly poor moist feeling. Reference Example 6 containing cellulose particles E manufactured using fine fibrous cellulose as a raw material without containing an additive had poor falling strength, slightly poor smoothness, a slightly poor moist feeling, and the like.

Reference Example 7 using cellulose powder E formed of an aggregate containing fine fibrous cellulose without containing an additive had improved falling strength, but poor smoothness and poor adhesion to the skin. Note that the glycerin contained in Reference Example 7 is added as one component of a moisturizing agent to the above-described mixture as an intermediate product, and does not constitute an aggregate.

### Industrial Applicability

The present invention can be used as a cosmetic such as a base, a face powder, a foundation, or a base makeup cosmetic.

## Claims

1. A solid powder cosmetic comprising
a cellulose powder having an average particle size of 1 to 25 µm and an aspect ratio of 0.6 to 0.9,
wherein the cellulose powder is an aggregate containing an additive and fine fibrous cellulose having an average fiber width of 1 to 500 nm.

2. The solid powder cosmetic according to claim 1,
wherein the cellulose powder is formed by aggregation of the fine fibrous cellulose.

3. The solid powder cosmetic according to claim 1, comprising:
60 to 99.9% by mass of a powder containing the cellulose powder; and 0.1 to 40% by mass of an oil component.

4. The solid powder cosmetic according to claim 1,
wherein the additive is glycerin.

5. The solid powder cosmetic according to claim 1,
wherein the cellulose powder has a moisture content of 1 to 15% in an atmosphere having a humidity of 50%.

6. The solid powder cosmetic according to claim 1,
wherein the cellulose powder has a bulk specific gravity of 0.5 g/cm³ or less.

7. The solid powder cosmetic according to claim 1,
wherein the cellulose powder has a specific surface area of 1.0 m²/g or more.

8. The solid powder cosmetic according to claim 1, comprising
1 to 99% by mass of the cellulose powder.

9. The solid powder cosmetic according to claim 1,
wherein the cellulose powder has a cellulose pulp viscosity of 1.0 to 7.0 mPa•s.

10. The solid powder cosmetic according to claim 1, which is a base makeup cosmetic.
